# EUROPEAN PATENT SPECIFICATION

(11) **EP 2 517 009 B1**
(45) Date of publication and mention of the grant of the patent: **06.11.2013**
(21) Application number: 10805669.8
(22) Date of filing: 22.12.2010
(51) Int. Cl.: G01N 33/50

(54) **METHOD FOR ASSESSING WATER RESISTANCE OF AN ANTIPERSPIRANT PRODUCT**
VERFAHREN ZUR BEURTEILUNG DER WASSERFESTIGKEIT EINES SCHWEISSHEMMENDEN PRODUKTS
PROCÉDÉ PERMETTANT D'ÉVALUER LA RÉSISTANCE À L'EAU D'UN PRODUIT ANTI-TRANSPIRANT

(30) Priority: 23.12.2009 US 289665 P
(43) Date of publication of application: 31.10.2012
(73) Proprietor: Colgate-Palmolive Company, New York, NY 10022 (US)
(72) Inventor: MORTILLO, Susan, Wyckoff New Jersey 07481 (US); KOZUBAL, Cheryl, Somerset New Jersey 08873 (US); JOGUN, Suzanne, Wayne New Jersey 07470 (US)
(74) Representative: Jenkins, Peter David
(86) International application number: PCT/US2010/061700
(87) International publication number: WO 2011/079161

(56) References cited:
- anonymous: "Guidelines for effectiveness testing for OTC antiperpirant drug products", Internet , 10 July 2009 (2009-07-10), pages 1-10, XP002628649, Retrieved from the Internet: URL:http://www.fda.gov/downloads/aboutFDA/ CentersOffices/CDER/ucm106437.pdf [retrieved on 2011-03-17] cited in the application
- LEROY D ET AL: "Sunscreen substantivity: comparison between water and sweat resistance tests", PHOTODERMATOLOGY, MUNKSGAARD, COPENHAGEN, DK, vol. 5, no. 1, 1 February 1988 (1988-02-01), pages 49-50, XP008134343, ISSN: 0108-9684

## Description

### BACKGROUND

For antiperspirant products, their efficacy can be measured using the procedure from the U.S. Food and Drug Administration, which is described in 21 CFR 350 and its subparts and in Guidelines for Effectiveness Testing of OTC Antiperspirant Drug Products (http://www.fda.gov/downloads/AboutFDA/CentersOffices/CDER/ucm106437.pdf). This method describes how to apply an antiperspirant to a test subject and have the test subject sit in a hot room to measure the level of sweat. The level of sweat reduction is compared to an area on the test subject where no antiperspirant was applied or a control formulation was applied.

When people go swimming or to the beach, people may be concerned about how well their antiperspirant performs after being immersed in water. Up to now, there has been no method of assessing the water resistance of an antiperspirant product.

D. Leroy and A Dompmartin disclose, in Photodermatology, 1988, 5, 49-50, "Sunscreen substantivity: Comparison between water and sweat resistance tests".

### BRIEF SUMMARY

The present invention provides a method according to claim 1 for assessing water resistance of an antiperspirant comprising:
a) selecting a number of subjects;
b) conditioning the subjects by not using any products or using non-antiperspirant containing products in axillae for a conditioning period of time;
c) cleansing axillae of each subject,
d) applying a desired amount of an antiperspirant product to one axilla and a placebo to the other axilla on each subject;
e) conducting step d) until a desired number of applications are completed if more than one application is selected;
f) after the last application is completed, conducting a water challenge comprising having the subjects move around by performing at a moderate activity level in a pool of water of sufficient depth such that the axillae are wetted during the moving for an activity period of time and/or swim for an activity period of time in a pool of water of sufficient depth such that the axillae are wetted during the swimming;
g) conducting a sweat evaluation to determine a sweat amount for each axilla of each subject after the water challenge; and
h) determining if the antiperspirant product has at least standard antiperspirant efficacy.

Preferred features are defined in the dependent claims.

### DETAILED DESCRIPTION

In the event of a conflict in a definition in the present disclosure and that of a cited reference, the present disclosure controls.

The method is used to assess the water resistance of an antiperspirant product that has been applied to a subject. The method can be used with any form of antiperspirant product, such as sticks, soft solids, gels, aerosols, and roll-ons.

The method needs subjects to participate in a study. In certain embodiments, a statistically relevant number of subjects is selected. The statistical relevant number can come from having conducted similar antiperspirant efficacy studies and reviewing the number of subjects that provided statistically relevant results. In certain embodiments, the minimum number of subjects is at least 10, 20, 30, 40, 50, or more In certain embodiments, at least 40 subjects are initially selected. This allows for some subjects to not complete the study for whatever reason. For sprays, typically 50 people are selected for statistical significance because sprays do not include aluminum-zirconium antiperspirant, which is used in sticks, gels, creams, or roll-ons. Sprays contain aluminum salts, such as aluminum chlorohydrate, which do not have the same level of antiperspirant efficacy as aluminum-zirconium salts.

The subjects should be conditioned for a sufficient period of time prior to the start of the study to assure the washout of any prior used antiperspirant. The time is selected such that there is no measurable amount of antiperspirant on the skin. In certain embodiments, the conditioning amount of time is at least 17 days.

Before applying the first application of antiperspirant product to be tested, the axillae are cleaned.

To one axilla, an antiperspirant product to be assessed is applied, and a placebo product (without antiperspirant active) is applied to the other axilla. The amount applied is selected to be a typical amount of antiperspirant product that a person would apply. Examples of typical amounts for different products are: 0.50g ± 0.02g (for a stick, gel, or soft solid product), 1.08g ± 0.05g (for an aerosol product), or 0.5 ml ± 0.02 ml (for a roll-on product). For consistency, the application area can be selected to be approximately a 10.2 x 15.2 cm (4x6 inch) area centered in the axillary vault.

The number of applications of the antiperspirant product can be selected. In one embodiment, there is one application. In other embodiments, the number can be selected to simulate continued use of the product. Even with washing on a daily basis, all traces of antiperspirant active may not be removed from the skin by washing. With continued use of a product, there can be a cumulative effect. The number of applications can be selected to be at least 2, 3, 4, 5, 6, 7, or 8. In certain embodiments, the number is at least 4, and in other embodiments, the number is at least 8. The time between applications can be selected to be one day to simulate daily use of the antiperspirant product.

After the last application, a water challenge is conducted. Subjects enter a pool of water and conduct moderate activity, such as moving around or some swimming. The pool of water is of sufficient depth such that the axillae of the subjects is wetted during the water challenge. In certain embodiments, the depth of the water is the depth of a typical swimming pool 1.2-1.5 meters (4-5 feet). The time in the water can be selected to be any amount of time that simulates typical activity. In certain embodiments, the time in the water is selected to be about 20 minutes. After 20 minutes, the subjects exit the pool. They can be permitted to towel dry off, but they should not disturb the area where the antiperspirant product was applied. If desired, the subjects can repeat the pool activity for any desired number of times. In one embodiment, the number is two. Between pool activities, there is a waiting period. In one embodiment, the waiting period is about 20 minutes. This can be used to simulate typical activity of being in the water, exiting the water, and reentering the water. There is no requirement for the conditions in the pool. Typically, the pool will be a public swimming pool with moderate pH (about 7) with typical chlorine levels (1-5 ppm) and normal temperature (about 30°C).

After the water challenge, the subjects are sent to a hot room for sweat collection. The period of time between the water challenge and the sweat evaluation can be selected to be any length of time. In one embodiment, the amount of time is about one hour. Any hot room procedure can be used. In one embodiment, the hot room procedure used is described below.

After sweat is collected from the hot room procedure for each subject, the water resistance of the antiperspirant product can be evaluated using standard statistical methods to determine if the antiperspirant product meets standard or enhanced levels of efficacy. Examples of statistical methods are provided below. If the antiperspirant product meets either of these levels of efficacy, then the antiperspirant product can be considered to be water resistant.

### SPECIFIC EMBODIMENTS

Below are non-limiting examples of various embodiments of the method.

Subjects are selected who are in good health. Factors for selecting subjects include, but are not limited to, persons should be 18-65 years old and in good general health. In certain embodiments, not more than 20% of the subjects should be over the age of 55. For good health, the subject should have a pulse ≤100, a body temperature ≤37.3°C (99.2°F), and blood press of ≤150 systolic and ≤90 diastolic. The subject must produce 100 mg of sweat per axilla during a 20 minute baseline sweat collection. A subject should be excluded from the study if: a) the subject has axillary irritation, b) the subject has a history of irritation or sensitivity to axillary antiperspirant, deodorant or soap products, c) the subject has a recurring history of infections, boils, abscesses, lymph node enlargement or lymph node removal in the axilla, d) the subject has active psoriasis, eczema, skin cancer or a dermatological condition that may interfere with the conduct of the study, e) the subject used systemic antibiotics or topical antibiotic medications in the axillary area within two weeks prior to the study, f) the subject is taking any medication or has a significant disease that may interfere with the study results or presents the subject as unhealthy for participation, g) the subject has the following condition(s): heart disease, uncontrolled hypertension, kidney disease, significant respiratory disease, epilepsy, heat intolerance, h) the subject has shaved the underarms within 48 hours of study start, or i) the subject is an insulin dependent diabetic.

Baseline sweat volumes can be determined to eliminate subjects who have perspiration levels less than 100 milligrams of sweat/20 minutes/axilla. Each subject abstains from the use of axillary antiperspirants during the entire baseline period. Baseline volumes can be used to compare differences between highest and lowest rates of sweating amongst the test subjects. In certain embodiments, the difference between the highest and lowest rate of sweat among the total group of test subjects must exceed 600 milligrams for either the right or left axilla during two 20-minute sweat collections. In one embodiment, subjects will be ranked in order of highest to lowest sweat output, such as from the right axilla. Subjects will be given a treatment assignment number. Subject 1 will be the highest sweater with subject 2 being the next highest sweater, and so on.

Before starting the study, the subject is conditioned to assure that the results of the study are based on the antiperspirant used in the study. Each subject will elect to use either a conditioning deodorant product (antiperspirant free) or nothing in the axillae for a sufficient period of time to affect washout of any previously used antiperspirant formulations. This should result in there being no measurable antiperspirant remaining in the axillae. Generally, the conditioning period is at least 17 days. Each subject is allowed ad libitum use of the conditioning deodorant during the conditioning period only and must abstain from the use of axillary antiperspirants during the entire conditioning period. In certain embodiments, the subjects' axilla are shaved 48 hours prior to the start of the study. This allows for the application of the antiperspirant product directly to the skin.

Supervised washes will be conducted prior to each test product application. The subjects will be instructed to wash according to the following procedure: 1) wash right axilla for 10 seconds using a disposable towel saturated with a 2% aqueous solution of Camay soap, 2) wet a fresh disposable towel under running water and rinse the axilla until all soap is removed, 3) gently pat dry the axilla using a dry disposable towel, and 4) repeat for left axilla.

For the study, one axilla will receive the product to be tested, and the other axilla will receive a placebo product. The distribution of which axilla receives the test product can be randomized among the subjects. During the study, no other axillary antiperspirant product will be used by any subject during the study. The effect of treatments on axillary sweating will be evaluated by sweat collections made at a specified interval following specified applications. Each subject will receive 4-8 treatment applications to the axillary areas, each 1 day apart. Test products will be applied at a rate of 0.50g ± 0.02g (for a stick product), 1.08g ± 0.05g (for an aerosol product), or 0.5 ml ± 0.02 ml (for a roll-on product) to uniformly cover approximately a 10.2 x 15.2 cm (4x6 inch) area centered in the axillary vault. For uniformity, all applications can be made by a technician. Subjects can wait at the test facility for approximately 20 minutes following each test product application to allow time for drying and absorption of test products.

Evaluations will be done at baseline and approximately 3 hours after the last treatment application (approximately 1 hour post water challenge).

Hot Room Testing - Sweating of the subjects is induced by having the subjects sit in a room maintained at a desired temperature and relative humidity. The temperature can be 36.8°C (98°F) to 55°C (131°F). The relative humidity can be 25 to 75%. In certain embodiments, the temperature can be 37 to 40°C. In certain embodiments, the relative humidity is 25 to 45. In one embodiment, the temperature is approximately 37.8°C ± 1°C (100°F ± 2°F), and the relative humidity is 35% ±5%.

During the first 40 minutes of the sweat stimulation period, the subjects hold unweighed non-woven cotton fabric pads (Webril™ pads from Kendall) in their axillae. This preliminary warm up period is followed by two successive 20-minute collection periods, during which the subjects hold weighed non-woven cotton fabric pads (Webril™ pads) in the axillae. These pads are weighed in tightly capped polystyrene vials before and after use. The vials are labeled with the subject's number, axilla and collection designation. The first collection made with weighed pads is designated Collection B and the second Collection C. During the collections with weighed pads, the subjects are required to sit in an erect position with both feet flat on the floor and with their arms resting against their sides in a symmetrical manner. The amount in grams of sweat collected is determined by subtracting the initial weight of the pad from the weight with the sweat.

All subjects will participate in a water challenge approximately 1 hour after the last treatment application. Subjects will be responsible for arriving at the pool after their test article application and a 20-minute wait. Subjects can be tested individually in the pool, or the subjects can participate as a group in the pool. Subjects will remain in the water for approximately 20 minutes. While in the water, subjects will be required to move around/swim at a moderate activity level. Subjects will exit the pool after 20 minutes and dry off making sure to avoid the underarm area. All subjects will enter the water again after sitting out for approximately 20 minutes. Subjects will stay in the water for an additional approximately 20 minutes performing at a moderate activity level. When approximately 20 minutes have elapsed, the subjects will dry off again (avoiding the underarms) and return to the lab to participate in the hot room testing approximately 1 hour later.

Antiperspirant activity can be evaluated by determining shifts in ratios of the sweat output by the treated axilla to the output of the untreated axilla for each panelist. Estimates of percent reduction and 95% confidence intervals will be calculated. The data can be analyzed using the Wilcoxon Signed Rank Test. The source data for this analysis are treated to control ratios adjusted for the ratio of right-to-left axillary sweating rates. These ratios are calculated using the post-treatment average B and C collections for each individual at each time period. The adjusted treated to control ratios for this analysis can be calculated as follows: Z = (PC x T) / (PT x C), where Z is the adjusted ratio, PC is the pretreatment measure of moisture for the control axilla (placebo), PT is the pretreatment measure for the test axilla, T is the treated measure for the test axilla, and C is the corresponding quantity for the control axilla (placebo).

The following can be used to demonstrate Standard Antiperspirant Efficacy. The study results are analyzed by comparing the adjusted ratio to 0.80, the ratio which corresponds to a 20 percent reduction in moisture due to treatment. The hypothesis that reduction in perspiration exceeds 20 percent is tested statistically by subtracting 0.80 from Z for all subjects and testing the resulting number with the Wilcoxon signed rank test. The hypotheses tested in the signed rank test are stated below:
Hₒ: Median Z ≥0.80
Hₐ: Median Z < 0.80
Hypothesis testing will be performed at the a = 0.05 level. Rejection of the null hypothesis will justify the conclusion that at least 50 percent of the target population will obtain a sweat reduction of at least 20 percent.

The following can be used to demonstrate Extra Effective Antiperspirant Efficacy. The study results are analyzed by comparing the adjusted ratio to 0.70, the ratio which corresponds to a 30 percent reduction in moisture due to treatment. The hypothesis that reduction in perspiration exceeds 30 percent is tested statistically by subtracting 0.70 from Z for all subjects and testing the resulting number with the Wilcoxon signed rank test. The hypotheses tested in the signed rank test are stated below:
Hₒ: Median Z ≤ 0.70
Hₐ: Median Z < 0.70
Hypothesis testing will be performed at the α = 0.05 level. Rejection of the null hypothesis will justify the conclusion that at least 50 percent of the target population will obtain a sweat reduction of at least 30 percent.

If, after the water challenge, the antiperspirant product meets the level of standard antiperspirant efficacy or extra effective antiperspirant efficacy, the antiperspirant product can be considered water resistant.

## Claims

1. A method for assessing water resistance of an antiperspirant comprising:
a) selecting a number of subjects;
b) conditioning the subjects by not using any products or using non-antiperspirant containing products in axillae for a conditioning period of time;
c) cleansing axillae of each subject,
d) applying a desired amount of an antiperspirant product to one axilla and a placebo to the other axilla on each subject;
e) conducting step d) until a desired number of applications are completed if more than one application is selected;
f) after the last application is completed, conducting a water challenge comprising having the subjects move around by performing at a moderate activity level in a pool of water of sufficient depth such that the axillae are wetted during the moving for an activity period of time and/or swim for an activity period of time in a pool of water of sufficient depth such that the axillae are wetted during the swimming;
g) conducting a sweat evaluation; and
h) determining if the antiperspirant product has at least standard antiperspirant efficacy.

2. The method of claim 1, wherein the number of subjects is at least 30.

3. The method of any preceding claim, wherein the period of time for conditioning is at least 17 days.

4. The method of any preceding claim, wherein the number of applications is at least 4.

5. The method of any preceding claim, wherein the activity period of time is 20 minutes.

6. The method of any preceding claim, wherein there is one hour between finishing the water challenge and conducting the final sweat evaluation.

7. The method of any preceding claim, wherein the water challenge further comprises having the subjects wait for a waiting period of time and then having the subjects move around or swim in the pool again for the activity period of time.

8. The method of claim 7, wherein the waiting period of time is 20 minutes.

9. The method of claim 1, wherein:
a) the number of subjects is at least 30;
b) the conditioning period of time is at least 17 days;
c) the number of applications is at least 4, and there is one day between applications;
d) the water challenge comprises
i) having the subjects move around or swim for an 20 minutes,
ii) having the subjects exit the pool and remain out of the pool for 20 minutes, and
iii) having the subjects move around or swim in the pool again for 20 minutes; and
e) the final sweat evaluation is conducted 1 hour after the water challenge.

## Patentansprüche

1. Verfahren zur Bestimmung der Wasserbeständigkeit eines Antitranspirants, bei dem:
a) eine Zahl an Testpersonen ausgewählt wird;
b) die Testpersonen konditioniert werden, indem keine Produkte oder nicht Antitranspirant enthaltende Produkte in den Achselhöhlen für einen Konditionierungszeitraum verwendet werden;
c) die Achselhöhlen einer jeden Testperson gereinigt werden;
d) eine gewünschte Menge eines Antitranspirantprodukts in eine Achselhöhle und ein Placebo in die andere Achselhöhle jeder Testperson aufgetragen werden;
e) Schritt d) ausgeführt wird, bis eine gewünschte Anzahl an Anwendungen vervollständigt ist, wenn mehr als eine Anwendung ausgewählt wurde;
f) nachdem die letzte Anwendung vollständig ist, eine Wasseraufgabe durchgeführt wird, bei der die Testpersonen aufgefordert werden sich in einem Schwimmbecken mit Wasser von ausreichender Tiefe zu bewegen, indem sie eine moderate Beschäftigung ausüben, so dass die Achselhöhlen während der Bewegung für einen Beschäftigungszeitraum benässt werden und/oder in einem Schwimmbecken mit Wasser von ausreichender Tiefe zu schwimmen, so dass die Achselhöhlen während des Schwimmens benässt werden;
g) eine Schweißbeurteilung durchgeführt wird;
h) bestimmt wird, ob das Antitranspirantprodukt mindestens Standard-Antitranspirantwirkung aufweist.

2. Verfahren nach Anspruch 1, beim dem die Zahl der Testpersonen mindestens 30 ist.

3. Verfahren nach einem vorgehenden Anspruch, bei dem der Konditionierungszeitraum mindestens 17 Tage beträgt.

4. Verfahren nach einem vorgehenden Anspruch, bei dem die Zahl der Anwendungen mindestens 4 beträgt.

5. Verfahren nach einem vorgehenden Anspruch, bei dem der Beschäftigungszeitraum 20 Minuten beträgt.

6. Verfahren nach einem vorgehenden Anspruch, bei dem eine Stunde zwischen der Beendigung der Wasseraufgabe und der Durchführung der finalen Schweißbeurteilung liegt.

7. Verfahren nach einem vorgehenden Anspruch, bei dem die Wasseraufgabe weiterhin vorsieht, dass die Testpersonen eine Zeitspanne lang warten und sich dann wieder über den Beschäftigungszeitraum bewegen oder schwimmen.

8. Verfahren nach Anspruch 7, bei dem die Wartezeit 20 Minuten beträgt.

9. Verfahren nach Anspruch 1, bei dem
a) die Zahl der Testpersonen mindestens 30 beträgt;
b) der Konditionierungszeitraum mindestens 17 Tage beträgt;
c) die Zahl der Anwendungen mindestens 4 beträgt und ein Tag zwischen den Anwendungen liegt;
d) bei der Wasseraufgabe
i) die Testpersonen 20 Minuten sich bewegen oder schwimmen,
ii) die Testpersonen das Schwimmbecken verlassen und sich 20 Minuten außerhalb des Schwimmbeckens aufhalten,
iii) die Testpersonen 20 Minuten sich wieder bewegen oder wieder schwimmen; und
e) die finale Schweißbeurteilung eine Stunde nach der Wasseraufgabe stattfindet.

## Revendications

1. Procédé destiné à évaluer la résistance à l'eau d'un antitranspirant comprenant les étapes consistant à :
a) sélectionner un certain nombre de sujets ;
b) conditionner les sujets pour qu'ils n'utilisent aucun produit, ou uniquement des produits ne contenant pas d'antitranspirant, sous les aisselles pendant une période de conditionnement ;
c) nettoyer les aisselles de chaque sujet ;
d) appliquer une quantité souhaitée d'un produit antitranspirant sous une aisselle et un placebo sous l'autre aisselle de chaque sujet ;
e) exécuter l'étape d) jusqu'à ce qu'un nombre d'applications souhaité soit atteint si plusieurs applications sont sélectionnées ;
f) une fois la dernière application réalisée, effectuer un test à l'eau qui consiste en ce que les sujets se déplacent autour d'une piscine d'une profondeur suffisante en se livrant à un niveau d'activité modéré de telle sorte que les aisselles soient mouillées pendant une période d'activité au cours du déplacement, et/ou nagent dans une piscine d'une profondeur suffisante pendant une période d'activité de telle sorte que les aisselles soient mouillées au cours de la baignade ;
g) effectuer une évaluation de la transpiration ; et
h) déterminer si le produit antitranspirant présente au moins une efficacité antitranspirante standard.

2. Procédé selon la revendication 1, dans lequel le nombre de sujets est au moins égal à 30.

3. Procédé selon l'une quelconque des revendications précédentes, dans lequel la période de conditionnement est au moins égale à 17 jours.

4. Procédé selon l'une quelconque des revendications précédentes, dans lequel le nombre d'applications est au moins égal à 4.

5. Procédé selon l'une quelconque des revendications précédentes, dans lequel la période d'activité est égale à 20 minutes.

6. Procédé selon l'une quelconque des revendications précédentes, dans lequel il s'écoule une heure entre la fin du test à l'eau et l'exécution de l'évaluation de la transpiration finale.

7. Procédé selon l'une quelconque des revendications précédentes, dans lequel le test à l'eau prévoit en outre que les sujets observent une période d'attente, et ensuite que les sujets se déplacent autour de la piscine ou nagent dans celle-ci de nouveau pendant une seconde période d'activité.

8. Procédé selon la revendication 7, dans lequel la période d'attente est égale à 20 minutes.

9. Procédé selon la revendication 1, dans lequel :
a) le nombre de sujets est au moins égal à 30 ;
b) la période de conditionnement est au moins égale à 17 jours ;
c) le nombre d'applications est au moins égal à 4, et il s'écoule un jour entre chaque application ;
d) le test à l'eau comprend les étapes suivantes :
i) les sujets se déplacent autour de la piscine ou nagent dans celle-ci pendant 20 minutes ;
ii) les sujets sortent de la piscine et restent hors de la piscine pendant 20 minutes ; et
iii) les sujets se déplacent autour de la piscine ou nagent dans celle-ci de nouveau pendant 20 minutes ;
et
e) l'évaluation de la transpiration finale est effectuée une heure après le test à l'eau.
